# FASCICULE DE BREVET EUROPEEN

(11) **EP 1 814 847 B1**
(45) Date de publication et mention de la délivrance du brevet: **12.01.2011**
(21) Numéro de dépôt: 05818188.4
(22) Date de dépôt: 17.11.2005
(51) Int. Cl.: C07C 401/00, C07C 39/15, C07C 37/20

(54) **NOUVEAU PROCEDE DE PREPARATION DU 3-[5'-(3,4-BIS-HYDROXYMETHYL-BENZYLOXY)-2'- ETHYL-2-PROPYL-BIPHENYL-4-YL]-PENTA-3OL.**
NEUES VERFAHREN ZUR HERSTELLUNG VON 3-[5'-(3,4-BIS-HYDROXYMETHYL-BENZYLOXY)-2'- ETHYL-2-PROPYL-BIPHENYL-4-YL]-PENTA-3-OL
NOVEL METHOD FOR PREPARING 3-[5'-(3,4-BIS-HYDROXYMETHYL-BENZYLOXY)-2'- ETHYL-2-PROPYL-BIPHENYL-4-YL]-PENTA-3-OL

(30) Priorité: 19.11.2004 FR 0412325
(43) Date de publication de la demande: 08.08.2007
(73) Titulaire: Galderma Research & Development, 06410 Biot (FR)
(72) Inventeur: TERRANOVA, Eric, F-06520 Magagnosc (FR); DAVER, Sébastien, F-06600 Antibes (FR); MARTY, Christine, F-06410 Biot (FR); PASCAL, Jean-Claude, F-06100 Nice (FR)
(74) Mandataire: Allab, Myriam
(86) Numéro de dépôt international: PCT/FR2005/002856
(87) Numéro de publication internationale: WO 2006/053985

(56) Documents cités:
- WO-A-03/050067
- WO-A-2005/058918
- WO-A-2005/061520

## Description

La présente invention concerne un procédé de préparation du 3-[5'-(3,4-bis-hydroxymethyl-benzyloxy)-2'-ethyl-2-propyl-biphenyl-4-yl]-penta-3-ol de formule :

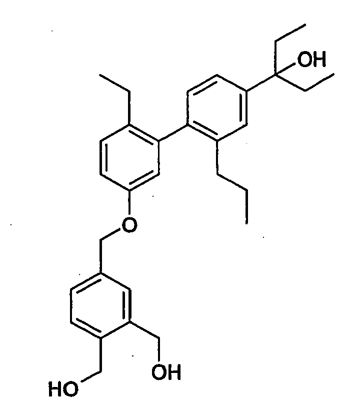

Les composés de la famille du 3-[5'-(3,4-bis-hydroxymethyl-benzyloxy)-2'-ethyl-2-propyl-biphenyl-4-yl]-penta-3-ol ci-dessus, et leur utilisation en médecine humaine ont été décrits par la demanderesse dans le brevet WO 03/050067.

Dans cette demande de brevet, la synthèse du 3-[5'-(3,4-bis-hydroxyméthylbenzyloxy)-2'-éthyl-2-propyl-biphényl-4-yl]-penta-3-ol est effectuée en 17 étapes. La plupart des intermédiaires générés dans cette synthèse sont purifiés par chromatographie sur colonne de silice rendant la fabrication de ce produit difficile à grande échelle.

Egalement, du fait de ce nombre important d'étapes, le rendement global de cette synthèse est très faible, inférieur à 0,5%, et les temps de fabrication sont très longs.

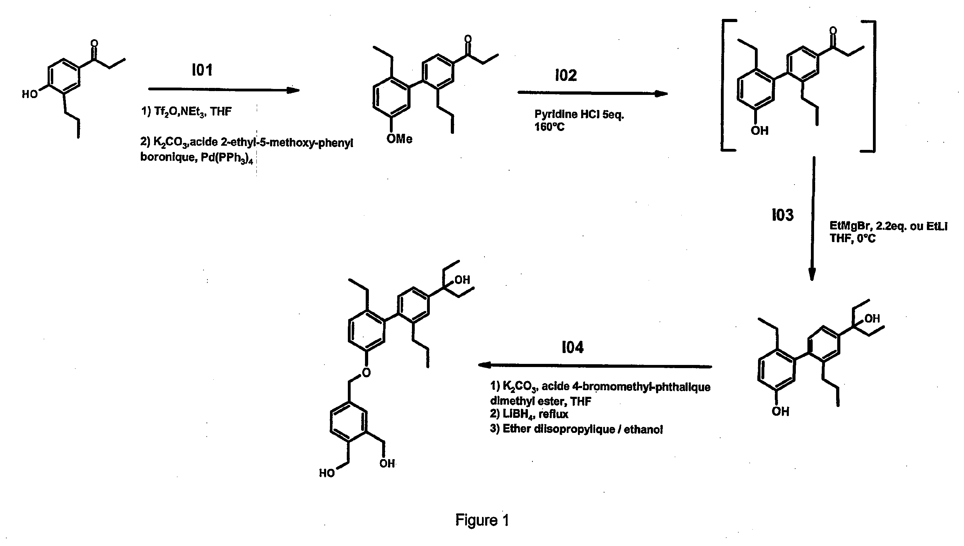

Dans la présente invention, la demanderesse a mis au point un nouveau procédé de préparation du 3-[5'-(3 ,4-bis-hydroxyméthyl-benzyloxy)-2'-éthyl-2-propyl-biphényl-4-yl]-penta-3-ol en quatre étapes permettant de remédier aux problèmes exposés ci-dessus selon le schéma de la figure 1.

L'invention concerne donc un procédé de préparation du 3-[5'-(3,4-bis-hydroxyméthylbenzyloxy)-2'-éthyl-2-propyl-biphényl-4-yl]-penta-3-ol **caractérisé en ce qu'**il comprend les étapes suivantes :
a) conversion de la 1-(4-hydroxy-3-propyl-phenyl)-propan-1-one en acide trifluorométhane sulfonique 4-propionyl-2-n-propyl-phényl ester suivie, *in situ,* d'une réaction de type Suzuki avec l'acide 2-éthyl-5-méthoxy-phényl-boronique ;
b) déméthylation de la 1-(2'-ethyl-5'-methoxy-2-propyl-biphenyl-4-yl)-propane-1-one par chauffage avec un excès de sels de pyridine ;
c) conversion de la 1-(2'-éthyl-5'-hydroxy-2-propyl-biphényl-4-yl)-propane-1-one en 6-éthyl-4'-(1-éthyl-1-hydroxy-propyl)-2'-propyl-biphenyl-3-ol par réaction avec du bromure d'éthyle magnésium ou de l'éthyle lithium ;
d) condensation du 6-éthyl-4'-(1-éthyl-1-hydroxy-propyl)-2'-propyl-biphenyl-3-ol avec l'acide 4-bromométhyl-phthalique, diméthyl ester suivie, *in situ,* d'une réaction de réduction avec du borohydrure de lithium.

De façon plus précise, le procédé selon l'invention comprend les étapes détaillées suivantes :
- La première étape de ce procédé (étape a) ci-dessus ou 101 sur la figure 1) est une réaction « one pot » de conversion de la 1-(4-hydroxy-3-propyl-phenyl)-propan-1-one (préparé selon Demerseman et al. Bull.Soc.Chim.Fr.; 1963 ; 2559-2562 ou Stoughton ; Baltzly ; Bass. J.Am.Chem.Soc. ;56 ;1934 ;2007) par action de l'anhydride trifluorométhane sulfonique (Tf₂O) en présence de triéthylamine (NEt₃) en son dérivé acide trifluorométhane sulfonique 4-propionyl-2-n-propyl-phenyl ester suivie d'une condensation, *in situ,* de type Suzuki avec l'acide 2-éthyl-5-méthoxy-phényl boronique (préparé selon un procédé décrit dans la demande de brevet FR2863613 en présence de K₂CO₃ et d'une quantité catalytique de PdCl₂(PPh₃)₂ ou de Pd(PPh₃)₄.

Cette réaction « one pot » s'effectue dans des solvants tels que le tétrahydrofurane (THF), le diméthylformamide (DMF), les solvants aromatiques comme le toluène, les solvants éthérés comme le diisopropyléther, les solvants halogénés comme le chloroforme, les alcanes comme le pentane, l'hexane ou l'heptane. Les solvants préférentiellement utilisés dans cette réaction sont le DMF et/ou le toluène. La réaction s'effectue à des températures comprises entre 5 et 140°C préférentiellement à 120°C.

Les quantités de catalyseur PdCl₂(PPh₃)₂ ou Pd(PPh₃)₄ utilisés dans cette réaction peuvent varier entre 0.001 et 0.05 équivalent molaire par rapport à la quantité de 1-(4-hydroxy-3-propyl-phenyl)-propan-1-one. De préférence, on utilisera entre 0.01 et 0.05 équivalent molaire de PdCl₂(PPh₃)₂ ou de Pd(PPh₃)₄.
- Dans la seconde étape (étape b) ci-dessus ou 102 sur la figure 1), la 1-(2'-éthyl-5'-méthoxy-2-propyl-biphényl-4-yl)-propane-1-one est soumise à une réaction de déméthylation avec un sel de pyridine. La réaction s'effectue sans solvant à des températures comprises entre 80 et 200°C préférentiellement à 170°C.

Les sels de pyridine utilisés dans cette réaction peuvent être le chlorhydrate, le bromhydrate ou l'iodhydrate et peuvent varier entre 1 et 10 équivalents molaires. De préférence on utilisera 5 équivalents molaires.
- La troisième étape (étape c) ci-dessus ou 103 sur la figure 1) est la transformation du 1-(2'-éthyl-5'-hydroxy-2-propyl-biphényl-4-yl)-propane-1-one en un composé nouveau, le 6-éthyl-4'-(1-éthyl-1-hydroxy-propyl)-2'-propyl-biphenyl-3-ol par réaction avec le bromure d'éthyle magnésium ou avec l'éthyle lithium.

Les solvants préférentiellement utilisés dans cette réaction sont des éthers tels que l'éther éthylique, le terbutyl diméthyl éther ou le tetrahydrofurane.

La réaction s'effectue à des températures comprises entre -20°C et 20°C, préférentiellement à 0°C.

Les quantités de bromure d'éthyle magnésium ou d'éthyle lithium utilisées dans cette réaction peuvent varier entre 2 et 5 équivalents molaires. De préférence on utilisera 2.2 équivalents molaires.
- Dans l'étape suivante (étape d) ci-dessus ou 104 sur la figure 1), le 6-éthyl-4'-(1-éthyl-1-hydroxy-propyl)-2'-propyl-biphényl-3-ol est condensé avec l'acide 4-bromométhyl-phthalique di-méthyl ester (préparé selon une méthode analogue à celle décrit par E.H.White, D.F.Roswell et O.C.Zafiriou dans J.Org.Chem. 34 (8), 2462-2468, 1969 et J.W.Leon, M.Kawa et J.M.J.Frechet dans J.Amer.Chem.Soc., 118,8847-8859,1996) en présence de carbonate de potassium (K₂CO₃) dans le tétrahydrofurane au reflux. Les deux fonctions carboxyliques sont ensuite réduites *in situ* par l'addition de borohydrure de lithium (LiBH₄) puis chauffage au reflux du tétrahydrofurane.

La première réaction peut être catalysée par des agents de transfert de phase tels que l'Aliquat 336 ou le bromure de tetrabutyl ammonium en présence d'iodure de potassium.

La présente invention concerne également le composé nouveau 6-éthyl-4'-(1-éthyl-1-hydroxy-propyl)-2'-propyl-biphenyl-3-ol de structure : 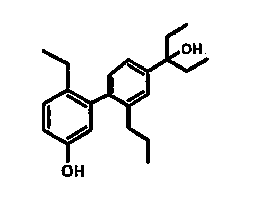 et son procédé de fabrication.

L'invention concerne donc également le procédé de transformation du 1-(2'-éthyl-5'-hydroxy-2-propyl-biphényl-4-yl)-propane-1-one en 6-éthyl-4'-(1-éthyl-1-hydroxy-propyl)-2'-propyl-biphenyl-3-ol par réaction avec le bromure d'éthyle magnésium ou avec de l'éthyle lithium.

Les solvants préférentiellement utilisés dans cette réaction sont des éthers tels que l'éther éthylique, le terbutyl diméthyl éther ou le tetrahydrofurane.

La réaction s'effectue à des températures comprises entre -20°C et 20°C, préférentiellement à 0°C. Les quantités de bromure d'éthyle magnésium ou d'éthyle lithium utilisées dans cette réaction peuvent varier entre 2 et 5 équivalents molaires. De préférence on utilisera 2.2 équivalents molaires.

Après traitement le 6-éthyl-4'-(1-éthyl-1-hydroxy-propyl)-2'-propyl-biphenyl-3-ol est cristallisé dans du diisopropyl ether ou du dichlorométhane permettant d'obtenir ce produit avec une pureté supérieur à 99%.

L'invention se rapporte également à l'utilisation du 6-éthyl-4'-(1-éthyl-1-hydroxypropyl)-2'-propyl-biphenyl-3-ol pour la préparation du 3-[5'-(3 ,4-bis-hydroxymethylbenzyloxy)-2'-ethyl-2-propyl-biphenyl-4-yl]-penta-3-ol.

A titre d'exemple, sans aucun caractère limitatif, on trouvera ci-dessous une description du procédé permettant la préparation du 3-[5'-(3,4-bis-hydroxymethylbenzyloxy)-2'-ethyl-2-propyl-biphenyl-4-yl]-penta-3-ol.

### Exemple 1: Préparation 3-[5'-(3.4-bis-hydroxymethyl-benzyloxy)-2'-ethyl-2-propyl-biphenyl-4-yl]-penta-3-ol.

### 1) 1-(2'-ethyl-5'-methoxy-2-propyl-biphenyl-4-yl)-propane-1-one

Dans un réacteur de 4 litres sous azote, on charge 203g de 1-(4-hydroxy-3-propylphenyl)-propan-1-one et 1 litre de toluène. On refroidit le milieu vers -5°C puis on additionne rapidement 176mL de triéthylamine, puis 196mL d'anhydride trifluoromethane sulfonique en 1 heure entre -5°C et +1 °C. Après 30mn d'agitation, on introduit 1 litre d'une solution K₂CO₃ 2M, puis 190g d'acide 2-ethyl-5-methoxy-phenylboronique en solution dans 610mL de diméthylformamide. On ajoute 12g de tetrakis triphenylphosphine palladium (0) et on chauffe le milieu réactionnel au reflux pendant 2h. Après être revenu à température ambiante, on lave le milieu réactionnel 3 fois avec 610mL d'une solution NH₄Cl saturée puis avec 610mL d'eau. On évapore sous vide les solvants de la phase organique. On reprend le produit brut obtenu dans 1 volume de dichlorométhane que l'on dépose sur 3 fois son poids de silice. On élue avec 16 volumes de chlorure de mèthylène. Après évaporation du solvant, on obtient 335g de 1-(2'-ethyl-5'-hydroxy-2-propyl-biphenyl-4-yl)-propan-1-one (huile beige ; rendement 100%).

### 2) 1-(2'-éthyl-5'-hydroxy-2-propyl-biphényl-4-y/)-propane-1-one

Dans un ballon on place 413 g (1.33 moles) de 1-(2'-ethyl-5'-methoxy-2-propylbiphenyl-4-yl)-propane-1-one et 768 g (6.64 moles) de chlorhydrate de pyridine. Sous agitation le mélange est chauffé à 160-170°C pendant 4 heures. On laisse le milieu réactionnel revenir à 100-110°C et on ajoute 800 ml d'eau. Le mélange est refroidi à 30°C et extrait avec 1,6 litres d'acétate d'éthyle. Après décantation, la phase aqueuse est extraite avec 600 ml d'acétate d'éthyle. Les phases organiques sont combinées et lavées deux fois avec 800 ml d'eau. Après évaporation sous vide réduit, on obtient 410g de 1-(2'-éthyl-5'-hydroxy-2-propyl-biphényl-4-yl)-propane-1-one sous forme d'une huile.

### 3) 6-éthyl-4-(1-éthyl-1-hydroxy-propyl)-2'-propyl-biphenyl3-ol

Dans un réacteur on place 370g (1.248 moles) de 1-(2'-éthyl-5'-hydroxy-2-propylbiphényl-4-yl)-propane-1-one en solution dans 3.7 litres de tétrahydrofurane. A cette solution refroidie à -10°C, on coule lentement 915 ml d'une solution 3M de bromure d'éthyle magnésium dans l'éther éthylique. A la fin de l'addition, le mélange réactionnel est maintenu sous agitation pendant 1 heure puis transféré sur 5 litres d'une solution de chlorure d'ammonium 2.5 molaire dans l'eau. La phase organique est décantée, lavée deux fois avec 800 ml d'eau. Après évaporation sous vide réduit, le résidu est dissous par 2.75 litres de chlorure de méthylène au reflux. On laisse le milieu revenir à température ambiante sous agitation puis on le refroidit à 5°C. Les cristaux sont filtrés puis séchés sous vide. On obtient 260g (64%) de 6-éthyl-4'-(1-éthyl-1-hydroxy-propyl)-2'-propyl-biphenyl-3-ol.
Point de fusion : 130°C
¹H RMN (DMSO d₆) (ppm): 0.67-0.74, m; 9H, 0.9, t, 3H; 1.38, m,2H;1.71-1.77, m,4H ;2.13-2.35, m,4H ; 4.48,s,1H;6.45 ,d , 1H, 6.70, dd,1H; 6.94,d,1H; 7.07,d,1H; 7.18,dd, 1H; 7.26,s,1H; 9.16,s,1H

### 4) 13-[5'-(3,4-bis-hydroxymethyl-benzyloxy)-2'-ethyl-2 Propyl-biphenyl-]-penta-3-ol

Dans un ballon muni d'une agitation mécanique, on introduit 40g (0.123 mole) de 6-éthyl-4'-(1-éthyl-1-hydroxy-propyl)-2'-propyl-biphenyl-3-ol, 37 g (0.129 mole) d'acide 4-bromo-méthyl-phthalique, diméthyl ester, 17.8 g (0.129 mole) de carbonate de potassium, 500 mg d'Aliquat 336 et 100 mg d'iodure de potassium dans 400 ml de tetrahydrofurane. Le mélange réactionnel est chauffé au reflux pendant 6 heures. Après retour à température ambiante, on ajoute par portions 4 g (0.184 mole) de borohydrure de lithium. On chauffe à nouveau pendant 4 heures. On laisse le milieu revenir à température ambiante puis on le transfère lentement sur 600 ml d'eau glacée. Après deux heures sous agitation la phase organique est extraite par 100 ml d'acétate d'éthyle, lavée deux fois avec 200 ml d'eau. La phase organique est évaporée sous vide réduit et le résidu est dissous dans un mélange éther diisopropylique/éthanol à 50°C.

Après une nuit sous agitation, les cristaux sont filtrés et séchés sous vide. On obtient 41.6 g (71%) de 3-[5'-(3,4-bis-hydroxymethyl-benzyloxy)-2'-ethyl-2-propyl-biphenyl-]-penta-3-ol.

## Revendications

1. Procédé de préparation du 3-[5'-(3,4-bis-hydroxymethyl-benzyloxy)-2'-ethyl-2-propyl-biphenyl-4-yl]-penta-3-ol de structure : 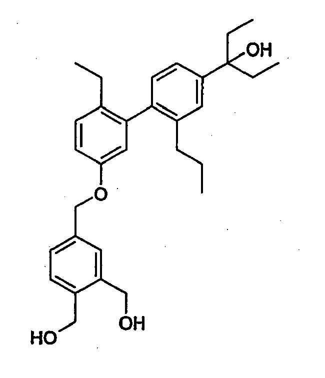 **caractérisé en ce qu'**il comprend les 4 étapes suivantes :
a) conversion de la 1-(4-hydroxy-3-propyl-phenyl)-propan-1-one en acide trifluorométhane sulfonique 4-propionyl-2-n-propyl-phényl ester suivie d'une réaction avec l'acide 2-éthyl-5-méthoxy-phényl-boronique ;
b) déméthylation de la 1-(2'-ethyl-5'-methoxy-2-propyl-biphenyl-4-yl)-propane-1-one par chauffage avec un excès de sels de pyridine ;
c) conversion de la 1-(2'-éthyl-5'-hydroxy-2-propyl-biphényl-4-yl)-propane-1-one en 6-éthyl-4'-(1-éthyl-1-hydroxy-propyl)-2'-propyl-biphenyl-3-ol par réaction avec du bromure d'éthyle magnésium ou avec de l'éthyle lithium ;
d) condensation du 6-éthyl-4'-(1-éthyl-1-hydroxy-propyl)-2'-propylbiphenyl-3-ol avec l'acide 4-bromo-méthyl-phthalique, diméthyl ester suivie, *in situ,* d'une réaction de réduction avec du borohydrure de lithium.

2. Procédé selon la revendication 1, **caractérisé en ce que** la réaction de l'étape a) est une réaction de conversion de la 1-(4-hydroxy-3-propyl-phenyl)-propan-1-one par action de l'anhydride trifluorométhane sulfonique (Tf₂O) en présence de triéthylamine (NEt₃) en son dérivé acide trifluorométhane sulfonique 4-propionyl-2-n-propyl-phenyl ester, suivie d'une condensation de type Suzuki avec l'acide 2-éthyl-5-méthoxy-phényl boronique et **en ce que** lesdites réactions de l'étape a) se font *in situ* en présence de K₂CO₃, d'une quantité catalytique de PdCl₂(PPh₃)₂ ou de Pd(PPh₃)₄, dans des solvants tels que le tétrahydrofurane (THF), le diméthylformamide (DMF), les solvants aromatiques comme le toluène, les solvants éthérés comme le diisopropyléther, les solvants halogénés comme le chloroforme, les alcanes comme le pentane, l'hexane ou l'heptane.

3. Procédé selon la revendication 1 ou 2, **caractérisé en ce que** la réaction de l'étape a) est réalisée à des températures comprises entre 5 et 140°C et que les quantités catalytiques de PdCl₂(PPh₃)₂ ou de Pd(PPh₃)₄ sont comprises entre 0.01 et 0.05 équivalent molaire.

4. Procédé selon l'une quelconque des revendications 1 à 3, **caractérisé en ce que** le sel de pyridine de l'étape b) est choisi parmi un chlorhydrate, bromhydrate ou iodhydrate de pyridine à une concentration pouvant varier entre 1 et 10 équivalents molaires.

5. Procédé selon l'une quelconque des revendications 1 à 4, **caractérisé en ce que** le sel de pyridine de l'étape b) est utilisé à une concentration de 5 équivalents molaires.

6. Procédé selon l'une quelconque des revendications 1 à 5, **caractérisé en ce que** l'étape b) est réalisée sans solvant.

7. Procédé selon l'une quelconque des revendications 1 à 6, **caractérisé en ce que** l'étape b) est réalisée à des températures comprises entre 80 et 200°C.

8. Procédé selon l'une quelconque des revendications 1 à 7, **caractérisé en ce que** l'étape b) est réalisée à la température de 170°C.

9. Procédé selon l'une quelconque des revendications 1 à 8, **caractérisé en ce que** l'étape c) est la transformation du 1-(2'-éthyl-5'-hydroxy-2-propyl-biphényl-4-yl)-propane-1-one en 6-éthyl-4'-(1-éthyl-1-hydroxy-propyl)-2'-propyl-biphenyl-3-ol par réaction avec le bromure d'éthyle magnésium ou avec l'éthyle lithium, en présence de solvants tels que l'éther éthylique, le terbutyl diméthyl éther ou le tetrahydrofurane.

10. Procédé selon l'une quelconque des revendications 1 à 9, **caractérisé en ce que** l'étape d) est la condensation du 6-éthyl-4'-(1-éthyl-1-hydroxy-propyl)-2'-propylbiphényl-3-ol avec l'acide 4-bromométhyl-phthalique di-méthyl ester en présence de carbonate de potassium (K₂CO₃) dans le tétrahydrofurane au reflux. Les deux fonctions carboxyliques sont ensuite réduites *in situ* par l'addition de borohydrure de lithium (LiBH₄) puis chauffage au reflux du tétrahydrofurane.

11. Composé 6-éthyl-4'-(1-éthyl-1-hydroxy-propyl)-2'-propyl-biphenyl-3-ol de structure :

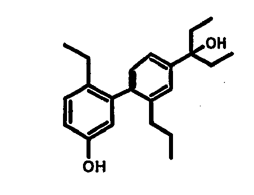

12. Procédé de préparation du 6-éthyl-4'-(1-éthyl-1-hydroxy-propyl)-2'-propylbiphenyl-3-ol de structure : 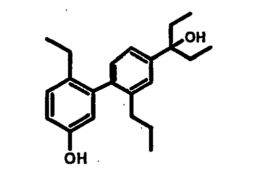 **caractérisé en ce que** le composé est obtenu à partir de la 1-(2'-éthyl-5'-hydroxy-2-propyl-biphényl-4-yl)-propane-1-one par addition de bromure d'éthyle magnésium ou d'éthyle lithium en présence de solvants.

13. Procédé selon la revendication 12, **caractérisé en ce que** les solvants utilisés sont des éthers tels que l'éther éthylique, le terbutyl diméthyl éther ou le tetrahydrofurane.

14. Procédé selon la revendication 12 ou 13 précédentes, **caractérisé en ce que** la réaction s'effectue à des températures comprises entre -20°C et 20°C.

15. Procédé selon l'une quelconque des revendications 12 à 14 précédentes, **caractérisé en ce que** la réaction s'effectue à la température de 0°C.

16. Procédé selon l'une quelconque des revendications 12 à 15 précédentes, **caractérisé en ce que** les quantités de bromure d'éthyle magnésium ou d'éthyle lithium peuvent varier entre 2 et 5 équivalents molaires.

17. Procédé selon l'une quelconque des revendications 12 à 16 précédentes, **caractérisé en ce que** les quantités de bromure d'éthyle magnésium sont 2.2 équivalents molaires.

18. Utilisation du 6-éthyl-4'-(1-éthyl-1-hydroxy-propyl)-2'-propyl-biphenyl-3-ol de structure : 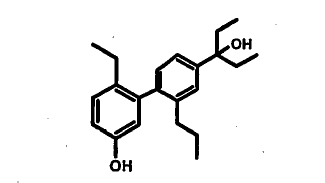 pour la préparation du 3-[5'-(3,4-bis-hydroxymethyl-benzyloxy)-2'-ethyl-2-propylbiphenyl-4-yl]-penta-3-ol.

## Claims

1. Process for the preparation of 3-[5'-(3,4-bis(hydroxymethyl)benzyloxy)-2'-ethyl-2-propylbiphenyl-4-yl]pentan-3-ol with the structure: 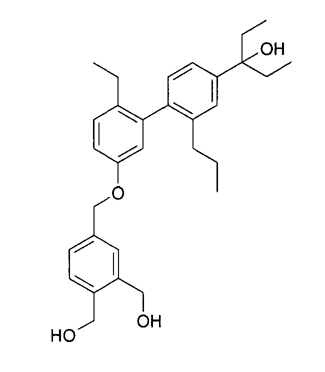 **characterized in that** it comprises the following 4 stages:
a) conversion of 1-(4-hydroxy-3-propylphenyl)propan-1-one to give trifluoromethanesulphonic acid 4-propionyl-2-(n-propyl)phenyl ester, followed by a reaction with 2-ethyl-5-methoxyphenylboronic acid;
b) demethylation of 1-(2'-ethyl-5'-methoxy-2-propylbiphenyl-4-yl)propan-1-one by heating with an excess of pyridine salts;
c) conversion of 1-(2'-ethyl-5'-hydroxy-2-propylbiphenyl-4-yl)propan-1-one to give 6-ethyl-4'-(1-ethyl-1-hydroxypropyl)-2'-propylbiphenyl-3-ol by reaction with ethylmagnesium bromide or with ethyllithium;
d) condensation of 6-ethyl-4'-(1-ethyl-1-hydroxypropyl)-2'-propylbiphenyl-3-ol with dimethyl 4-(bromomethyl)phthalate, followed *in situ* by a reduction reaction with lithium borohydride.

2. Process according to Claim 1, **characterized in that** the reaction of stage a) is a reaction for the conversion of 1-(4-hydroxy-3-propylphenyl)propan-1-one by reaction with trifluoromethanesulphonic anhydride (Tf₂O) in the presence of triethylamine (NEt₃) to give its derivative trifluoromethanesulphonic acid 4-propionyl-2-(n-propyl)phenyl ester, followed by a condensation of Suzuki type with 2-ethyl-5-methoxyphenylboronic acid, and **in that** the said reactions of stage a) take place *in situ* in the presence of K₂CO₃ and of a catalytic amount of PdCl₂(PPh₃)₂ or Pd(PPh₃)₄, in solvents such as tetrahydrofuran (THF), dimethylformamide (DMF), aromatic solvents, such as toluene, ethereal solvents, such as diisopropyl ether, halogenated solvents, such as chloroform, or alkanes, such as pentane, hexane or heptane.

3. Process according to Claim 1 or 2, **characterized in that** the reaction of stage a) is carried out at temperatures of between 5 and 140°C and that the catalytic amounts of PdCl₂(PPh₃)₂ or Pd(PPh₃)₄ are between 0.01 and 0.05 molar equivalent.

4. Process according to any one of Claims 1 to 3, **characterized in that** the pyridine salt of stage b) is chosen from a pyridine hydrochloride, hydrobromide or hydriodide at a concentration which can vary between 1 and 10 molar equivalents.

5. Process according to any one of Claims 1 to 4, **characterized in that** the pyridine salt of stage b) is used at a concentration of 5 molar equivalents.

6. Process according to any one of Claims 1 to 5, **characterized in that** stage b) is carried out without solvent.

7. Process according to any one of Claims 1 to 6, **characterized in that** stage b) is carried out at temperatures of between 80 and 200°C.

8. Process according to any one of Claims 1 to 7, **characterized in that** stage b) is carried out at a temperature of 170°C.

9. Process according to any one of Claims 1 to 8, **characterized in that** stage c) is the conversion of 1-(2'-ethyl-5'-hydroxy-2-propylbiphenyl-4-yl)propan-1-one to give 6-ethyl-4'-(1-ethyl-1-hydroxypropyl)-2'-propylbiphenyl-3-ol by reaction with ethylmagnesium bromide or with ethyllithium in the presence of solvents, such as ethyl ether, tert-butyl dimethyl ether or tetrahydrofuran.

10. Process according to any one of Claims 1 to 9, **characterized in that** stage d) is the condensation of 6-ethyl-4'-(1-ethyl-1-hydroxypropyl)-2'-propylbiphenyl-3-ol with dimethyl 4-(bromomethyl)phthalate in the presence of potassium carbonate (K₂CO₃) in tetrahydrofuran at reflux. The two carboxyl functional groups are subsequently reduced *in situ* by the addition of lithium borohydride (LiBH₄) and then heating at reflux of the tetrahydrofuran.

11. Compound 6-ethyl-4'-(1-ethyl-1-hydroxypropyl)-2'-propylbiphenyl-3-ol with the structure:

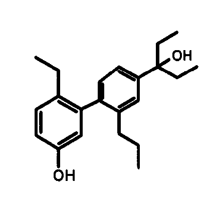

12. Process for the preparation of 6-ethyl-4'-(1-ethyl-1-hydroxypropyl)-2'-propylbiphenyl-3-ol with the structure: 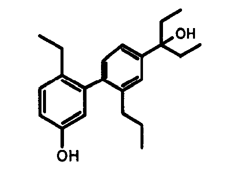 **characterized in that** the compound is obtained from 1-(2'-ethyl-5'-hydroxy-2-propylbiphenyl-4-yl)propan-1-one by addition of ethylmagnesium bromide or of ethyllithium in the presence of solvents.

13. Process according to Claim 12, **characterized in that** the solvents used are ethers, such as ethyl ether, tert-butyl dimethyl ether or tetrahydrofuran.

14. Process according to the preceding Claim 12 or the preceding Claim 13, **characterized in that** the reaction is carried out at temperatures of between -20°C and 20°C.

15. Process according to any one of the preceding Claims 12 to 14, **characterized in that** the reaction is carried out at the temperature 0°C.

16. Process according to any one of the preceding Claims 12 to 15, **characterized in that** the amounts of ethylmagnesium bromide or of ethyllithium can vary between 2 and 5 molar equivalents.

17. Process according to any one of the preceding Claims 12 to 16, **characterized in that** the amounts of ethylmagnesium bromide are 2.2 molar equivalents.

18. Use of 6-ethyl-4'-(1-ethyl-1-hydroxypropyl)-2'-propylbiphenyl-3-ol with the structure: 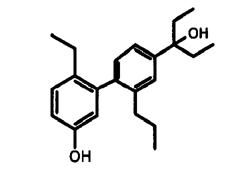 in the preparation of 3-[5'-(3,4-bis(hydroxymethyl)benzyloxy)-2'-ethyl-2-propylbiphenyl-4-yl]pentan-3-ol.

## Patentansprüche

1. Verfahren zur Herstellung von 3-[5'-(3,4-Bis-hydroxymethylbenzyloxy)-2'-ethyl-2-propyl-biphenyl-4-yl]-penta-3-ol der Struktur: 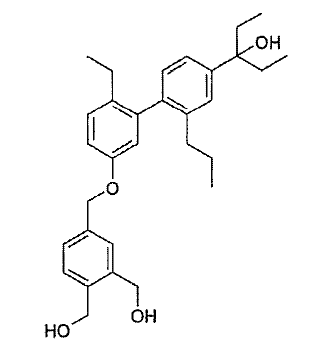 **dadurch gekennzeichnet, dass** es die folgenden 4 Schritte umfasst: a) Umwandlung des 1-(4-Hydroxy-3-propyl-phenyl)-propan-1-ons in den Trifluormethansulfonsäure-4-propionyl-2-n-propyl-phenylester und nachfolgende Umsetzung vom Suzuki-Typ mit 2-Ethyl-5-methoxy-phenyl-boronsäure;
b) Entmethylierung des 1-(2'-Ethyl-5'-methoxy-2-propyl-biphenyl-4-yl)-propan-1-ons durch Erwärmen mit einem Überschuss an Pyridinsalzen;
c) Umwandlung des 1-(2'-Ethyl-5'-hydroxy-2-propyl-biphenyl-4-yl)-propan-1-ons in das 6-Ethyl-4'-(1-ethyl-1-hydroxy-propyl)-2'-propyl-biphenyl-3-ol durch Umsetzung mit Ethylmagnesiumbromid oder Ethyllithium;
d) Kondensation des 6-Ethyl-4'-(1-ethyl-1-hydroxy-propyl)-2'-propylbiphenyl-3-ols mit 4-Brommethyl-phthalsäuredimethylester, gefolgt von einer Reduktion mit Lithiumborhydrid in situ.

2. Verfahren nach Anspruch 1, **dadurch gekennzeichnet, dass** es sich bei der Umsetzung des Schritts a) um eine Umwandlung des 1-(4-Hydroxy-3-propylphenyl)-propan-1-ons in sein Trifluormethansulfonsäure-4-propionyl-2-n-propylphenylesterderivat durch Einwirkung von Trifluormethansulfonsäure (Tf₂O) in Gegenwart von Triethylamin (NEt₃) und anschließende Kondensation vom Suzuki-Typ mit 2-Ethyl-5-methoxy-phenylboronsäure handelt und **dadurch**, dass die Reaktionen des Schritts a) in situ in Gegenwart von K₂CO₃ und einer katalytischen Menge von PdCl₂(PPh₃)₂ oder Pd(PPh₃)₄ in Lösemitteln wie Tetrahydrofuran (THF), Dimethylformamid (DMF), aromatischen Lösemitteln wie Toluol, Etherlösemitteln wie Diisopropylether, halogenierten Lösemitteln wie Chloroform, Alkanen wie Pentan, Hexan oder Heptan ablaufen.

3. Verfahren nach Anspruch 1 oder 2, **dadurch gekennzeichnet, dass** die Umsetzung des Schritts a) bei Temperaturen im Bereich von 5 bis 140 °C durchgeführt wird und **dadurch**, dass die katalytischen Mengen von PdCl₂(PPh₃)₂ oder Pd(PPh₃)₄ im Bereich von 0,01 bis 0,05 Moläquivalenten liegen.

4. Verfahren nach einem der Ansprüche 1 bis 3, **dadurch gekennzeichnet, dass** das Pyridinsalz in Schritt b) unter PyridinHydrochlorid, Pyridin-Hydrobromid oder Pyridin-Hydroiodid in einer Konzentration, die im Bereich von 1 bis 10 Moläquivalenten liegen kann, ausgewählt ist.

5. Verfahren nach einem der Ansprüche 1 bis 4, **dadurch gekennzeichnet, dass** das Pyridinsalz in Schritt b) in einer Konzentration von 5 Moläquivalenten verwendet wird.

6. Verfahren nach einem der Ansprüche 1 bis 5, **dadurch gekennzeichnet, dass** der Schritt b) ohne Lösemittel durchgeführt wird.

7. Verfahren nach einem der Ansprüche 1 bis 6, **dadurch gekennzeichnet, dass** der Schritt b) bei Temperaturen im Bereich von 80 bis 200 °C durchgeführt wird.

8. Verfahren nach einem der Ansprüche 1 bis 7, **dadurch gekennzeichnet, dass** der Schritt b) bei einer Temperatur von 170 °C durchgeführt wird.

9. Verfahren nach einem der Ansprüche 1 bis 8, **dadurch gekennzeichnet, dass** der Schritt c) eine Umformung des 1-(2'-Ethyl-5'-hydroxy-2-propyl-biphenyl-4-yl)- propan-1-ons in das 6-Ethyl-4'-(1-ethyl-1-hydroxypropyl)-2'-propyl-biphenyl-3-ol durch Umsetzung mit Ethylmagnesiumbromid oder mit Ethyllithium in Gegenwart von Lösemitteln wie Ethylether, tert-Butyldimethylether oder Tetrahydrofuran ist.

10. Verfahren nach einem der Ansprüche 1 bis 9, **dadurch gekennzeichnet, dass** es sich bei dem Schritt d) um die Kondensation des 6-Ethyl-4'-(1-ethyl-1-hydroxypropyl)-2'-propylbiphenyl-3-ols mit 4-Brommethylphthalsäuredimethylester in Gegenwart von Kaliumcarbonat (K₂CO₃) in Tetrahydrofuran bei Rückflußtemperatur handelt; die beiden Carboxyfunktionen werden dann in situ durch Zugabe von Lithiumborhydrid (LiBH₄) reduziert, wonach auf die Rückflußtemperatur des Tetrahydrofurans erwärmt wird.

11. 6-Ethyl-4'-(1-ethyl-1-hydroxy-propyl)-2'-propyl-biphenyl-3-ol der Struktur:

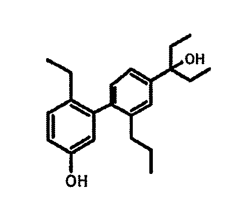

12. Verfahren zur Herstellung des 6-Ethyl-4'-(1-ethyl-1-hydroxypropyl)-2'-propyl- biphenyl-3-ols der Struktur: 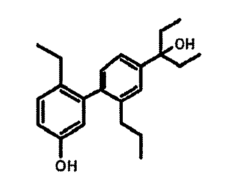 **dadurch gekennzeichnet, dass** die Verbindung ausgehend von 1-(2'-Ethyl-5'-hydroxy-2-propyl-biphenyl-4-yl)-propan-1-on durch Zugabe von Magnesiumethylbromid oder Ethyllithium in Gegenwart von Lösemitteln gebildet wird.

13. Verfahren nach Anspruch 12, **dadurch gekennzeichnet, dass** es sich bei den verwendeten Lösemitteln um Ether, wie Ethylether, tert-Butyldimethylether, oder Tetrahydrofuran handelt.

14. Verfahren nach Anspruch 12 oder 13, **dadurch gekennzeichnet, dass** die Umsetzung bei Temperaturen im Bereich von -20 bis 20 °C durchgeführt wird.

15. Verfahren nach einem der vorhergehenden Ansprüche 12 bis 14, **dadurch gekennzeichnet, dass** die Umsetzung bei einer Temperatur von 0 °C erfolgt.

16. Verfahren nach einem der vorhergehenden Ansprüche 12 bis 15, **dadurch gekennzeichnet, dass** die Mengenanteile des Magnesiumethylbromids oder Ethyllithiums im Bereich von 2 bis 5 Moläquivalenten liegen können.

17. Verfahren nach einem der vorhergehenden Ansprüche 12 bis 16, **dadurch gekennzeichnet, dass** die Mengenanteile des Magnesiumethylbromids 2,2 Moläquivalente sind.

18. Verwendung des 6-Ethyl-4'-(1-ethyl-1-hydroxy-propyl)-2'-propylbiphenyl-3-ols der Struktur: 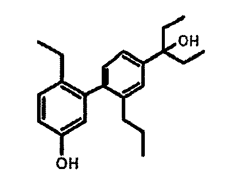 für die Herstellung des 3-[5'-(3,4-Bis-hydroxymethyl-benzyloxy)-2'-ethyl-2-propylbiphenyl-4-yl]-penta-3-ols.
